# EUROPEAN PATENT APPLICATION

(11) **EP 2 112 224 A2**
(43) Date of publication of application: **28.10.2009**
(21) Application number: 09164297.5
(22) Date of filing: 19.07.2006
(51) Int. Cl.: C12N 15/82, A01H 1/02, A01H 1/04, C12Q 1/68

(54) **Development of novel germplasm using segregates from transgenic crosses**

(30) Priority: 29.07.2005 US 703917 P
(62) Divisional of application: 06787991.6
(71) Applicant: Monsanto Technology, LLC, St. Louis, MO 63167 (US)
(72) Inventor: Arnevik, Cindy, Troy, MO 63379 (US); Dobert, Ray, O'Fallon, MO 63368 (US); Heck, Gregory, Crystal Lake Park, MO 63131 (US); Listello, Jennifer, O'Fallon, MO 63368 (US); Soteres, John, Olivette, MO 63132 (US); Wu, Kunsheng, Ballwin, MO 63021 (US); Zeng, Qingyi, Chesterfield, MO 63017 (US)
(74) Representative: Helbing, Jörg

(57) **Abstract**

This invention provides a method for the development of novel plant germplasm using segregates from a transgenic line combined with PCR-based zygosity testing and, optionally, Southern blot analysis.

## Description

### FIELD OF THE INVENTION

The invention in the field of plant breeding provides a method for the development of plant germplasm using segregates from a transgenic line. A method of zygosity testing to evaluate for the absence of a transgene is also disclosed.

### BACKGROUND OF THE INVENTION

Widely planted crop germplasm often represents the most elite lines containing a combination of the yield, agronomic, and pest and disease resistance traits most desired by growers. Because of its combination of elite traits, this germplasm may serve to generate commercially available seed, and may also be used as a source for future plant breeding efforts. In some cases, this germplasm may often comprise a transgenic trait in addition to the elite traits it exhibits. Thus, there exists a need in the field of plant breeding for methods to use the full complement of the existing germplasm base of a crop in plant breeding, regardless of whether a transgene is present in any of the elite varieties that might be used for breeding experiments. Methods to further apply zygosity testing to improve the efficiency of plant breeding efforts are also needed.

Zygosity and copy-number testing based on real-time quantitative TAQMAN^{™} PCR (qRT-PCR) has been used to analyze transgenic plant progeny (e.g. Bubner and Baldwin, Plant Cell Rep. 23:263-271 (2004)). This technique can allow detection and quantitation of the copy number of a given DNA sequence in a plant genome, and requires only a small amount of plant tissue. The technique is much less labor intensive per sample than previously employed methods, such as Southern blotting, for detecting and quantitating transgene copy numbers. Nevertheless, it is notoriously sensitive to the concentration and purity of the starting template DNA, among other variables. As such, its use has been primarily as a first screen for the copy number of a transgene before selective breeding is begun in earnest, with subsequent confirmation of results by other means such as Southern blotting. It is known to be suited, for instance, for identifying high copy number events that may be discarded early in a transgenic plant event analysis prior to selected events being passed on to breeders. The method has not been used with single copy elite transgenic breeding material.

If a transgenic plant line is to be used in a breeding program, efficient methods for removing a transgene, or screening for its loss, are desirable. Specific removal of transgene sequences, such as a gene encoding a selectable marker, has been reported, including the Cre/*lox* recombinase system (Hare and Chua, Nature Biotechnol. 20:575-580 (2002)). This method can specifically lead to removal of inserted transgenic DNA sequences from a transgenic plant, but *lox* site sequences necessarily still remain, flanking the site of the excised sequence. Screening for loss of a transgene by genetic segregation in progeny is another method that is widely known but requires substantial time and effort to achieve. Thus, more efficient methods to screen for loss of specific sequences are desirable, especially methods that ensure the complete removal of all inserted transgenic sequences.

A significant portion of recent soybean breeding has utilized lines containing the ROUNDUP READY® trait found in event 40-3-2, because possibly as much as 80-95% of the soybean germplasm offered for sale in the United States currently contains this transgenic event. This germplasm exhibits a set of agronomic traits superior to the non-transgenic A5403 parent line, and to many conventional (i.e. non-transgenic) soybean breeding lines. Thus these transgenic ROUNDUP READY® soybean varieties are themselves useful in plant breeding programs. In order to fully realize their usefulness, it is desirable to be able to identify progeny of these ROUNDUP READY® lines that retain the elite agronomic traits of the parental line except for glyphosate resistance derived from the transgene insert of event 40-3-2 and the associated transgenic sequences found in event 40-3-2.

The sequences of the functional transgene of soybean event 40-3-2 and its associated flanking plant DNA can be used to detect the presence of the transgene in progeny of 40-3-2. The DNA sequence flanking the functional transgenic insert in soybean event 40-3-2 has been characterized (Padgette et al. Crop Sci. 35:1451-1461 (1995); Windels et al. Eur. Food Res. Technol. 213:107-112 (2001).

Regulatory approval in many countries for release and commercialization of a particular transgenic plant event may require disclosure of genomic DNA sequences flanking an inserted DNA, and a method to detect DNA specific for the event. Accordingly, some sequences flanking the functional insertion in soybean event 40-3-2 have been reported previously (Monsanto MSL-16646, available at http link for //archive.food.gov.uk/pdf_files/ acnfp/dossier.pdf). A method of detection for corn comprising transgenic event NK.603 has also been reported at http link //gmo-crl.jrc.it/detectionmethods.htm.

### SUMMARY OF THE INVENTION

Plant breeders may utilize a set of "elite" cultivars exhibiting superior traits related to growth, adaptability, pest and disease resistance, seed yield, lodging resistance, emergence, maturity, late season plant intactness, plant height and shattering resistance among others, as a basis to develop new improved crop cultivars. These packages of traits can then be introduced into new breeding lines with one or more additional desired traits in order to efficiently improve the breeding germplasm. If the elite cultivar that is being used as a basis for further breeding already comprises a transgene, it is useful to be able to identify the presence or absence of the transgene among many segregating progeny while these progeny are being screened by classical plant breeding methods for their other agronomic qualities.

Plant breeders practicing the invention may use various techniques. Recurrent selection is a breeding procedure designed to accumulate favorable genes for a trait or traits in a population. Parent lines are crossed, their progeny are evaluated for one or more traits, and the progenies which best express the trait are intercrossed to repeat the process over successive generations. Variations on recurrent selection include, among others, full-sib selection, half-sib family selection, and S₁ progeny recurrent selection. Techniques such as backcross breeding, mass selection, and marker-assisted breeding may also be employed. These techniques may be used to select for traits of interest in self-pollinated and cross-pollinated crops, as appropriate.

This invention relates to, in part, a method for developing an elite crop variety, comprising crossing a first elite line exhibiting a useful trait and comprising a transgene that encodes the useful trait, and which exhibits additional elite traits or characteristics, with a second line that exhibits a useful trait; obtaining individual F1 hybrid lines; selecting at least one F1 hybrid individual that exhibits a useful trait from the first or second line; deriving at least one further progeny generation of seeds or plants from the selected F1 hybrid individual; screening F2 or later progeny exhibiting at least one of the additional elite traits or characteristics of the first line for the presence of the transgene; and identifying at least one individual lacking the trans gene, wherein the useful trait is derived from the second line.

In another aspect of the invention is a method of determining the zygosity of the hybrid progeny comprising: (a) contacting a sample comprising DNA derived from the progeny with a primer set comprising SEQ ID NO:3, SEQ ID NO:4 and SEQ ID NO:6, that when used in a nucleic-acid amplification reaction with genomic DNA from the progeny, produces a first amplicon that is diagnostic for the transgenic event; and (b) performing a nucleic acid amplification reaction, thereby producing the first amplicon; and (c) detecting the first amplicon; and (d) contacting the sample comprising progeny DNA with a primer set comprising SEQ ID NOs: 4, 5, and 7, that when used in a nucleic-acid amplification reaction with genomic DNA from progeny plants produces a second amplicon comprising the native genomic DNA homologous to the genomic region in which the transgene is inserted; and (e) performing a nucleic acid amplification reaction, thereby producing the second amplicon; and (f) detecting the second amplicon; and (g) comparing the first and second amplicons in a sample, wherein the presence of both amplicons indicates the sample is heterozygous for the transgene insertion.

In particular, the introduction of a new transgenic glyphosate tolerant trait into a soybean line already comprising event 40-3-2 would require the ability to distinguish between two events, since the application of glyphosate selection would not serve to identify the presence of the new event from the previous one. Additionally, if the new event comprises the same functional transgene as a previous event, ELISA technology would also be unable to distinguish the presence of either or both events. ELISA or other protein detection methods could allow a breeder to identify the presence of a novel glyphosate resistance trait in a line lacking any other copy of the transgene, but would not allow a breeder to evaluate in a positive manner whether any genetic elements attributed to event 40-3-2 still remain.

For evaluating and breeding traits that confer glyphosate tolerance either by themselves, or in vector stacks with other traits of value to producers, the use of DNA technology, in particular PCR zygosity technology based on flanking sequences for the 40-3-2 event will increase the efficiency of the selection process, and a breeder's ability to build upon the existing germplasm base that already contains event 40-3-2 as well as other useful traits. This is currently the only method that can demonstrate the absence of all identified genetic elements of the 40-3-2 event in breeding populations in a relatively inexpensive manner.

Corn breeding, for instance encompassing conversion of a corn line comprising a first event such as GA21 to a second event such as one comprising NK603, is also a subject of the present invention. Identification of a GA21-null segregant line that comprises event NK603 and lacks the presence of sequences associated with the GA21 event, following a cross between transgenic corn lines comprising events GA21 and NK603, is also a subject of the present invention.

In accordance with an aspect of the invention, mutagenesis can be used to create viable reproducible soybeans or other species of plant with unique genetic profiles starting with transgenic lines from which the transgene is to be removed. For example, mutagenesis of transgenic soybean or other species can be induced by treatment with a variety of mutagenic agents known in the art, including physical mutagens such as X-rays, gamma rays, fast or thermal neutrons, protons, and chemical mutagens such as ethyl methanesulfonate (EMS), diethyl sulfate (DES), ethyleneimine (EI), propane sultone, N-methyl-N-nitrosourethane (MNU), N-nitroso-N-methylurea (NMU), N-ethyl-N-nitrosourea (ENU) and sodium azide. For examples of these methods see Ohshima et al. (1998) Virology 243: 472-481; Okubara et al. (1994) Genetics 137: 867-874; Quesada et al. (2000) Genetics 154: 421-436. In addition, a fast and automatable method for screening for chemically induced mutations, TILLING (Targeting Induced Local Lesions In Genomes), using a denaturing HPLC or selective endonuclease digestion of selected PCR products is also applicable to the instant invention (see McCallum et al. (2000) Nat. Biotechnol. 18: 455-457). Genetic variants of soybeans can also be obtained, for example, by oligonucleotide-directed mutagenesis, linker-scanning mutagenesis, mutagenesis using the polymerase chain reaction, and the like. See, for example, Ausubel, pages 8.0.3-8.5.9. Also, see generally, McPherson (ed.), DIRECTED MUTAGENESIS: A Practical approach, (IRL Press, 1991). Following mutagenesis, the seeds are planted, evaluated and seeds of desirable novel genetic profiles are selected that are viable and capable of subsequent use. The resulting seeds can then be processed in accordance with the invention to remove the transgenic event and the resulting novel genetic profile progeny used in breeding and development programs.

This invention relates to a plant breeding method for identifying a transgenic plant, or cells or tissues thereof, which method is based on identifying the presence or absence of at least one transgenic DNA sequence. According to one aspect of the invention, the method for identifying in progeny of a transgenic plant, or cells or tissues thereof, comprises amplifying a sequence of a nucleic acid present in biological samples, using a polymerase chain reaction, with at least two primers, one of which recognizes, or hybridizes with, the plant DNA in the 5' or 3' flanking region of an insertion event, the other which recognizes a sequence within the inserted transgenic DNA. Preferably, the genomic DNA is analyzed according to the PCR identification protocol described here whereby one primer recognizes a sequence within the respective 5' or 3' flanking region comprising the nucleotide sequence of SEQ ID NO: 1 or SEQ ID NO:2 or its complement. Alternatively, the primers used may recognize a sequence within the insert comprising the nucleotide sequence of SEQ ID NO: 3, and the primer recognizing a sequence within the 3' flanking region comprises the nucleotide sequence of SEQ ID NO:5, so that at least one specific amplified fragment is detected.

This invention also relates to a method for producing a non-transgenic elite crop variety, comprising crossing an elite line, comprising a transgene and exhibiting one or more transgenically-derived elite traits and one or more conventionally-derived elite traits or characteristics, with a conventional non-transgenic line; obtaining individual F1 hybrid lines; selecting at least one F1 hybrid individual exhibiting elite characteristics of the elite line; deriving at least one further progeny generation of seeds or plants from the selected F1 hybrid individual; screening F2 or later progeny of the hybrid individual exhibiting the conventionally-derived elite traits or characteristics of the elite line for the presence of the transgene; and selecting progeny exhibiting the conventionally-derived elite traits or characteristics of the elite line wherein the transgene is absent from the progeny genome.

The invention also relates to a method for identifying null segregants for a transgene in a plant breeding program, comprising crossing a first line containing a transgene that encodes a useful trait inserted into its genome with a second line; obtaining F1 hybrid individuals; deriving at least one further progeny generation of seeds or plants from the F1 individual; performing zygosity analysis on the further progeny generation, and optionally Southern or western analyses; selecting progeny wherein elements of the transgene are absent and the insertion site of the transgene is restored to approximate its native state, and deriving further generations of the selected progeny wherein the removal of the transgene is not caused by the presence of a transgenic recombinase.

The present invention also relates to methods for identifying the presence or absence of DNA sequences in biological samples from progeny of a parent that comprises a transgene, the methods being based on primers or probes that specifically recognize the 5' and 3' plant DNA flanking sequence of the insert(s) of the transgenic event. Depending on the presence or absence of a transgene, an amplification product of a specific size can be detected.

The invention thus also relates to a kit for following the segregation of transgenic sequences in segregating progeny of an elite transgenic event, the kit comprising at least one primer or probe that specifically recognizes the 5' or 3' flanking region of the event, such that the presence, absence, or copy number of a given event may be followed during the plant breeding process.

Preferably the kit of the invention comprises, in addition to a primer that specifically recognizes the 5' or 3' flanking region of a given event, a second primer that specifically recognizes a sequence within the inserted DNA of the event, for use in a PCR identification protocol. Preferably, the kit of the invention comprises two (or more) specific primers, one of which recognizes a sequence within the 5' flanking region of an event, for instance a sequence within the plant DNA region of SEQ ID NO: 1 or SEQ ID NO:2, and another which recognizes a sequence within the inserted DNA. Especially preferably, the primer recognizing the plant DNA sequence within a 5' flanking region comprises the nucleotide sequence of SEQ ID NO:4, and the primer recognizing the inserted DNA comprises the nucleotide sequence of SEQ ID NO:3.

### DESCRIPTION OF THE DRAWINGS

The following figures form part of the present specification and are included to further demonstrate certain aspects of the present invention. The invention may be better understood by reference to one or more of these figures in combination with the detailed description of specific embodiments presented herein.
Figure 1 is a schematic map of 40-3-2 event and amplification result.

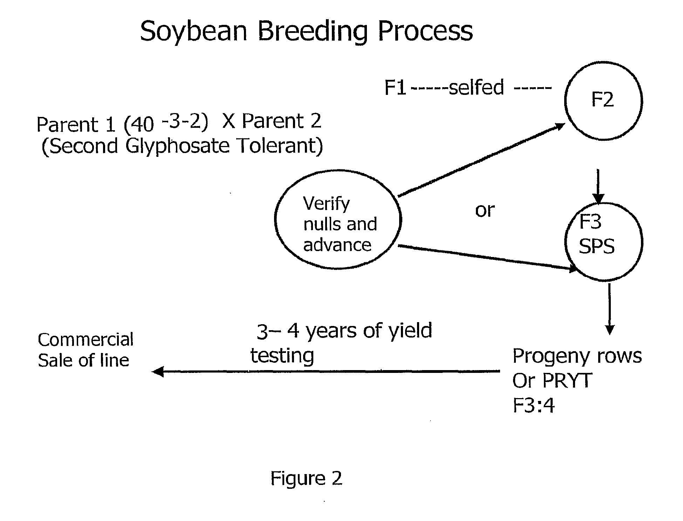

Figure 2 illustrates a soybean breeding process.

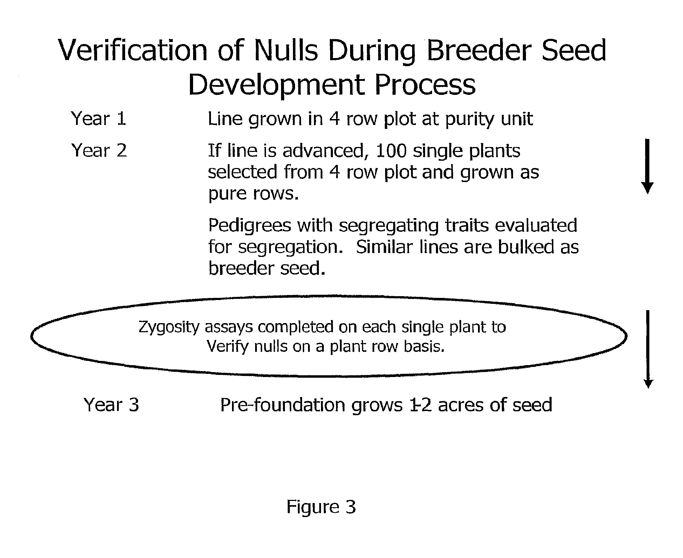

Figure 3 provides a verification scheme of nulls.

### DESCRIPTION OF THE SEQUENCE LISTINGS

The following sequence listings form part of the present specification and are included to further demonstrate certain aspects of the present invention. The invention may be better understood by reference to one or more of these sequences in combination with the detailed description of specific embodiments presented herein.

**Table 1**

| SEQ ID NO: | Description |
|---|---|
| 1 | 5' flanking genomic DNA and junction fragment of functional insert in 40-3-2 event |
| 2 | 3' flanking genomic DNA and junction fragment of functional insert in 40-3-2 event |
| 3 | SQ1147 primer; in transgene |
| 4 | SQ1148 primer; in 5' flanking genomic DNA |
| 5 | SQ3523 primer; in 3' flanking genomic DNA |
| 6 | PB65 fluorogenic primer; at 5' genomic-transgene junction |
| 7 | PB1172 fluorogenic primer; at 3' genomic-transgene junction |
| 8 | ract-F primer |
| 9 | ract-R primer |

### DEFINITIONS

The present invention is based, in part, on preserving elite germplasm by the identification of a genomic region comprising a transgene insert in the genome of a genetically modified crop first parent elite plant and the homologous region in the genome of a second parent plant of the same species not having the identical transgene insert, and utilizing DNA molecules in a DNA detection method to select progeny plants resulting from a cross of the parent plants, wherein the selected progeny plants do not contain the specific transgene insert of the first parent plant and contain some or all of the elite germplasm characteristics of the first parent. The following descriptions are provided to better define the present invention and to guide those of ordinary skill in the art in the practice of the present invention. Unless otherwise noted, terms are to be understood according to conventional usage by those of ordinary skill in the relevant art. Definitions of common terms in molecular biology may also be found in Rieger et al., Glossary of Genetics: Classical and Molecular, 5th edition, Springer-Verlag: New York, (1991); and Lewin, Genes V, Oxford University Press: New York, (1994). The nomenclature for DNA bases as set forth at 37 CFR § 1.822 is used. The standard one- and three-letter nomenclature for amino acid residues is used.

"DNA segment" refers to a DNA molecule that has been isolated free of total genomic DNA of a particular species.

The term "soybean" means "*Glycine max*" or soybean and includes all plant varieties that can be bred with soybean, including wild soybean species.

An "elite line" can refer, for example, to a crop genotype displaying one or more molecular or agronomic or quality or industrial traits of interest.

"Glyphosate" refers to N-phosphonomethylglycine and its' salts, Glyphosate is the active ingredient of ROUNDUP® herbicide (Monsanto Co.). Plant treatments with "glyphosate" refer to treatments with the ROUNDUP® or ROUNDUP ULTRA® herbicide formulation, unless otherwise stated. Glyphosate as N-phosphonomethylglycine and its' salts (not formulated ROUNDUP® herbicide) are components of synthetic culture media used for the selection of bacteria and plant tolerance to glyphosate or used to determine enzyme resistance in in vitro biochemical assays. Examples of commercial formulations of glyphosate include, without restriction, those sold by Monsanto Company as ROUNDUP®, ROUNDUP® ULTRA, ROUNDUP® ULTRAMAX, ROUNDUP® WEATHERMAX, ROUNDUP® CT, ROUNDUP® EXTRA, ROUNDUP® BIACTIVE, ROUNDUP® BIOFORCE, RODEO®, POLARIS®, SPARK® and ACCORD® herbicides, all of which contain glyphosate as its isopropylammonium salt; those sold by Monsanto Company as ROUNDUP® DRY and RIVAL® herbicides, which contain glyphosate as its ammonium salt; that sold by Monsanto Company as ROUNDUP® GEOFORCE, which contains glyphosate as its sodium salt; and that sold by Zeneca Limited as TOUCHDOWN® herbicide, which contains glyphosate as its trimethylsulfonium salt. Glyphosate herbicide formulations can be safely used over the top of glyphosate tolerant crops to control weeds in a field at rates as low as 8 ounces/acre upto 64 ounces/acre. Experimentally, glyphosate has been applied to glyphosate tolerant crops at rates as low as 4 ounces/acre and upto or exceeding 128 ounces/acre with no substantial damage to the crop plant.

The term "probe" is an isolated nucleic acid to which is attached a conventional detectable label or reporter molecule, e.g., a radioactive isotope, ligand, chemiluminescent agent, or enzyme. Such a probe is complementary to a strand of a target nucleic acid, in the case of the present invention, to a strand of genomic DNA from soybean event 40-3-2 whether from a soybean plant or from a sample that includes DNA from the event. Probes according to the present invention include not only deoxyribonucleic or ribonucleic acids but also polyamides and other probe materials that bind specifically to a target DNA sequence and can be used to detect the presence of that target DNA sequence.

The term "primer" refers to isolated nucleic acids that are annealed to a complementary target DNA strand by nucleic acid hybridization to form a hybrid between the primer and the target DNA strand, then extended along the target DNA strand by a polymerase, e.g., a DNA polymerase. Primer pairs of the present invention refer to their use for amplification of a target nucleic acid sequence, e.g., by the polymerase chain reaction (PCR) or other conventional nucleic-acid amplification methods.

The basis of this amplification method is multiple cycles of temperature changes to denature, then re-anneal the DNA primer molecules, followed by extension to synthesize new DNA strands in the region located between the flanking DNA primers. In general, DNA amplification can be accomplished by any of the various polynucleic acid amplification methods known in the art, including PCR. A variety of amplification methods are known in the art and are described, *inter alia*, in U.S. Patent Nos. 4,683,195 and 4,683,202 and in PCR Protocols: A Guide to Methods and Applications, ed. Innis et al., Academic Press, San Diego, 1990. PCR amplification methods have been developed to amplify up to 22 kb (kilobase) of genomic DNA and up to 42 kb of bacteriophage DNA (Cheng et al., Proc. Natl. Acad. Sci. USA 91:5695-5699, 1994). These methods, as well as other methods known in the art of DNA amplification may be used in the practice of the present invention.

The nucleic acid probes and primers of the present invention hybridize under stringent conditions to a target DNA sequence. Hybridization refers to the ability of a strand of nucleic acid to join with a complementary strand via base pairing. Hybridization occurs when complementary sequences in the two nucleic acid strands bind to one another. Nucleic acid molecules or fragments thereof are capable of specifically hybridizing to other nucleic acid molecules under certain circumstances. As used herein, two nucleic acid molecules are said to be capable of specifically hybridizing to one another if the two molecules are capable of forming an anti-parallel, double-stranded nucleic acid structure. A nucleic acid molecule is said to be the "complement" of another nucleic acid molecule if they exhibit complete complementarity. As used herein, molecules are said to exhibit "complete complementarity" when every nucleotide of one of the molecules is complementary to a nucleotide of the other. Two molecules are said to be "minimally complementary" if they can hybridize to one another with sufficient stability to permit them to remain annealed to one another under at least conventional "low-stringency" conditions. Similarly, the molecules are said to be "complementary" if they can hybridize to one another with sufficient stability to permit them to remain annealed to one another under conventional "high-stringency" conditions. Conventional stringency conditions are described by Sambrook *et al*., 1989, and by Haymes et al., In: Nucleic Acid Hybridization, A Practical Approach, IRL Press, Washington, DC (1985), hence forth referred to as Sambrook *et al*., 1989. Departures from complete complementarity are therefore permissible, as long as such departures do not completely preclude the capacity of the molecules to form a double-stranded structure. In order for a nucleic acid molecule to serve as a primer or probe it need only be sufficiently complementary in sequence to be able to form a stable double-stranded structure under the particular solvent and salt concentrations employed.

As used herein, a "substantially homologous DNA molecule" is a polynucleic acid molecule that will specifically hybridize to the complement of the polynucleic acid to which it is being compared under high stringency conditions. The term "stringent conditions" is functionally defined with regard to the hybridization of a nucleic-acid probe to a target nucleic acid (*i*.*e*., to a particular nucleic-acid sequence of interest) by the specific hybridization procedure discussed in Sambrook *et al*., 1989, at 9.52-9.55. *See also*, Sambrook *et al*., 1989 at 9.47-9.52, 9.56-9.58; Kanehisa, (Nucl. Acids Res. 12:203-213, 1984); and Wetmur and Davidson, (J. Mol. Biol. 31:349-370, 1988). Accordingly, the nucleotide sequences of the invention may be used for their ability to selectively form duplex molecules with complementary stretches of DNA fragments. Depending on the application envisioned, one will desire to employ varying conditions of hybridization to achieve varying degrees of selectivity of probe towards target sequence. For applications requiring high selectivity, one will typically desire to employ relatively high stringent conditions to form the hybrids, e.g., one will select relatively low salt and/or high temperature conditions, such as provided by about 0.02 M to about 0.15 M NaCl at temperatures of about 50°C to about 70°C. A high stringent condition, for example, is to wash the hybridization filter at least twice with high-stringency wash buffer (0.2X SSC, 0.1% SDS, 65° C). Appropriate moderate stringency conditions that promote DNA hybridization, for example, 6.0 x sodium chloride/sodium citrate (SSC) at about 45°C, followed by a wash of 2.0 x SSC at 50°C, are known to those skilled in the art or can be found in Current Protocols in Molecular Biology, John Wiley & Sons, N.Y. (1989), 6.3.1-6.3.6. Additionally, the salt concentration in the wash step can be selected from a low stringency of about 2.0 x SSC at 50°C to a high stringency of about 0.2 x SSC at 50°C. Additionally, the temperature in the wash step can be increased from low stringency conditions at room temperature, about 22°C, to high stringency conditions at about 65°C. Both temperature and salt may be varied, or either the temperature or the salt concentration may be held constant while the other variable is changed. Such selective conditions tolerate little mismatch between the probe and the template or target strand. Detection of DNA sequences via hybridization is well known to those of skill in the art, and the teachings of U.S. Patent Nos. 4,965,188 and 5,176,995 are exemplary of the methods of hybridization analyses.

The diagnostic amplicon produced by these methods may be detected by a plurality of techniques. One such method is Genetic Bit Analysis (Nikiforov, et al. Nucleic Acid Res. 22:4167-4175, 1994) where a DNA oligonucleotide is designed that overlaps both the adjacent flanking genomic DNA sequence and the inserted DNA sequence. The oligonucleotide is immobilized in wells of a microtiter plate. Following PCR of the region of interest (using one primer in the inserted sequence and one in the adjacent flanking genomic sequence), a single-stranded PCR product can be hybridized to the immobilized oligonucleotide and serve as a template for a single base extension reaction using a DNA polymerase and labelled dideoxynucleotide triphosphates (ddNTPs) specific for the expected next base. Readout may be fluorescent or ELISA-based. A signal indicates presence of the transgene/genomic sequence due to successful amplification, hybridization, and single base extension.

Another method is the Pyrosequencing technique as described by Winge (Innov. Pharma. Tech. 00:18-24, 2000). In this method an oligonucleotide is designed that overlaps the adjacent genomic DNA and insert DNA junction. The oligonucleotide is hybridized to single-stranded PCR product from the region of interest (one primer in the inserted sequence and one in the flanking genomic sequence) and incubated in the presence of a DNA polymerase, ATP, sulfurylase, luciferase, apyrase, adenosine 5' phosphosulfate and luciferin. DNTPs are added individually and the incorporation results in a light signal that is measured. A light signal indicates the presence of the transgene/genomic sequence due to successful amplification, hybridization, and single or multi-base extension.

Fluorescence Polarization as described by Chen, et al., (Genome Res. 9:492-498, 1999) is a method that can be used to detect the amplicon of the present invention. Using this method an oligonucleotide is designed that overlaps the genomic flanking and inserted DNA junction. The oligonucleotide is hybridized to single-stranded PCR product from the region of interest (one primer in the inserted DNA and one in the flanking genomic DNA sequence) and incubated in the presence of a DNA polymerase and a fluorescent-labeled ddNTP. Single base extension results in incorporation of the ddNTP. Incorporation can be measured as a change in polarization using a fluorometer. A change in polarization indicates the presence of the transgene/genomic sequence due to successful amplification, hybridization, and single base extension.

TAQMAN® (PE Applied Biosystems, Foster City, CA) is described as a method of detecting and quantifying the presence of a DNA sequence and is fully understood in the instructions provided by the manufacturer. Briefly, a FRET oligonucleotide probe is designed that overlaps the genomic flanking and insert DNA junction. The FRET probe and PCR primers (one primer in the insert DNA sequence and one in the flanking genomic sequence) are cycled in the presence of a thermostable polymerase and dNTPs. Hybridization of the FRET probe results in cleavage and release of the fluorescent moiety away from the quenching moiety on the FRET probe. A fluorescent signal indicates the presence of the transgene/genomic sequence due to successful amplification and hybridization.

Molecular Beacons have been described for use in sequence detection as described in Tyangi, et al. (Nature Biotech.14:303-308, 1996). Briefly, a FRET oligonucleotide probe is designed that overlaps the flanking genomic and insert DNA junction. The unique structure of the FRET probe results in it containing secondary structure that keeps the fluorescent and quenching moieties in close proximity. The FRET probe and PCR primers (one primer in the insert DNA sequence and one in the flanking genomic sequence) are cycled in the presence of a thermostable polymerase and dNTPs. Following successful PCR amplification, hybridization of the FRET probe to the target sequence results in the removal of the probe secondary structure and spatial separation of the fluorescent and quenching moieties. A fluorescent signal results. A fluorescent signal indicates the presence of the flanking/transgene insert sequence due to successful amplification and hybridization.

### ILLUSTRATIVE EMBODIMENTS OF THE INVENTION

Transgenic crops for which the method of the present invention can be applied include, but are not limited to herbicide tolerant crops, for example, ROUNDUP READY® Cotton 1445 and 88913; ROUNDUP READY® corn GA21, NK603, MON802, MON809; ROUNDUP READY® Sugarbeet GTSB77 and H7-1; ROUNDUP READY® Canola RT73 and GT200; oilseed rape ZSR500, ROUNDUP READY® Soybean 40-3-2, ROUNDUP READY® Bentgrass ASR368, HCN10, HCN28 and HCN92 canola, MS1 and RF1 canola, OXY-235 canola, PHY14, PHY35 and PHY36 canola, RM3-3, RM3-4 and RM3-6 chicory, A2704-12, A2704-21, A5547-35, A5547-127 soybean, GU262 soybean, W62 and W98 soybean, 19-51A cotton, 31807 and 31808 cotton, BXN cotton, FP967 flax, LLRICE06 and LLRICE62 rice, MON71800 wheat, 676 and 678 and 680 corn, B16 corn, Bt11 corn, CBH-351 corn, DAS-06275-8 corn, DBT418 corn, MS3 and MS6 com, T14 and T25 corn, H177 corn, and TC1507 corn.

Herbicides for which transgenic plant tolerance has been demonstrated and the method of the present invention can be applied, include but are not limited to: glyphosate, glufosinate, sulfonylureas, imidazolinones, bromoxynil, dalapon, cyclohezanedione, protoporphyrinogen oxidase inhibitors, and isoxaflutole herbicides. Polynucleotide molecules encoding proteins involved in herbicide tolerance are known in the art, and include, but are not limited to, a polynucleotide molecule encoding 5-enolpyruvylshikimate-3-phosphate synthase (EPSPS) described in U.S. Patent No. 5,627,061, U.S. Patent No. 5,633,435, U.S. Patent No. 6,040,497 and in U.S. Patent No. 5,094,945 for glyphosate tolerance, all of which are hereby incorporated by reference; polynucleotides encoding a glyphosate oxidoreductase and a glyphosate-N-acetyl transferase (GOX, U.S. Patent 5,463,175 and GAT, U.S. Patent publication 20030083480, herein incorporated by reference); a polynucleotide molecule encoding bromoxynil nitrilase (*Bxn*) described in U.S. Patent No. 4,810,648 for Bromoxynil tolerance, which is hereby incorporated by reference; a polynucleotide molecule encoding phytoene desaturase (*crtI*) described in Misawa et al, (1993) Plant J. 4:833-840 and Misawa et al, (1994) Plant J. 6:481-489 for norflurazon tolerance; a polynucleotide molecule encoding acetohydroxyacid synthase (AHAS, *aka* ALS) described in Sathasiivan et al. (1990) Nucl. Acids Res. 18:2188-2193 for tolerance to sulfonylurea herbicides; and the *bar* gene described in DeBlock, et al. (1987) EMBO J. 6:2513-2519 for glufosinate and bialaphos tolerance; resistant hydroxyphenyl pyruvate dehydrogenase (HPPD, U.S. Patent 6,768,044). The promoter of the present invention can express genes that encode for phosphinothricin acetyltransferase, glyphosate resistant EPSPS, aminoglycoside phosphotransferase, hydroxyphenyl pyruvate dehydrogenase, hygromycin phosphotransferase, neomycin phosphotransferase, dalapon dehalogenase, bromoxynil resistant nitrilase, anthranilate synthase, glyphosate oxidoreductase and glyphosate-N-acetyl transferase.

Transgenic crops for which the method of the present invention can be applied to include, but are not limited to, insect resistant crops, for example, cotton events, such as MON15985, 281-24-236, 3006-210-23, MON531, MON757, MON1076, and COT102; or corn events, such as 176, BT11, CBH-351, DAS-06275-8, DBT418, MON80100, MON810, MON863, TC1507, MIR152V, 3210M, and 3243M.

The following examples are included to demonstrate preferred embodiments of the invention. It should be appreciated by those of skill in the art that the techniques disclosed in the examples which follow represent techniques discovered by the inventors to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

### EXAMPLES

### Example 1: Progeny of 40-3-2 lacking inserted DNA

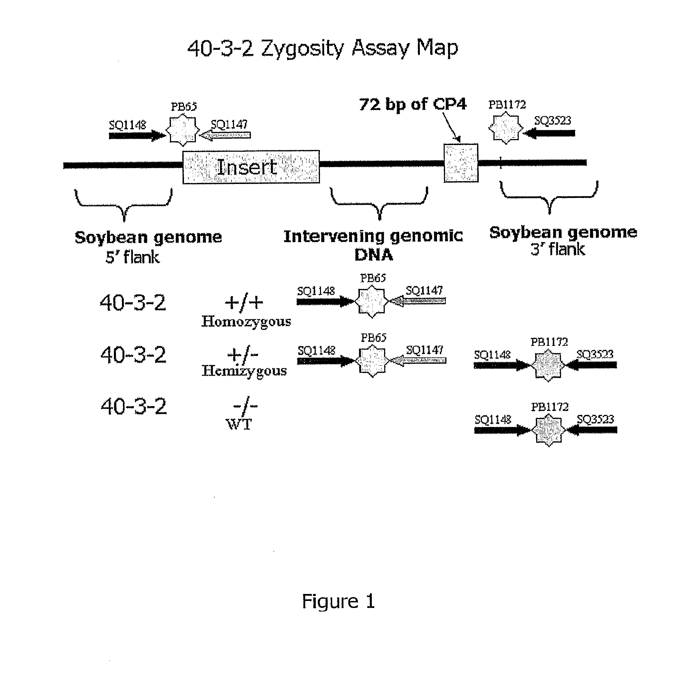

An elite soybean cultivar comprising ROUNDUP READY® event 40-3-2 is crossed with a conventional (non-transgenic) cultivar, and segregation of traits and the transgene is followed by PCR zygosity testing. The presence of 5' or 3' junction sequences may be followed as shown schematically in Figure 1. The PCR assay may be either singleplex, or multiplex. An internal quantitation control may be included. In the case of the 40-3-2 event, the inserted DNA consists of, in the 5' to 3' direction, a functional insert, an intervening (rearranged) genomic DNA, a non-functional 72 bp fragment of the gene encoding CP4 EPSPS, and additional genomic sequence. Referring to Figure 1, use of primer set A-B-C on template DNA derived from progeny of a cross of soybean event 40-3-2 with another plant, for instance, allows one to positively differentiate between progeny that are homozygous for the inserted DNA, hemizygous for the inserted DNA, or lack the inserted DNA. PCR primer design based on known DNA sequences is well known in the art. Exemplary primers in this example include SEQ ID NO:3 (primer B), SEQ ID NO:4 (primer A), and SEQ ID NO:5 (primer C).

Template DNA is extracted from plant leaf tissues or ground seed from progeny of the cross noted above. Briefly, a 7-mm hole punch may be used to harvest tissue of a newly formed leaf of a young plant (less than 1 month old). The tissue is lyophilized and stored in a tube until needed. Three 3mm glass beads are added to the tissue, and the tube is agitated to grind the tissue into a fine powder. 600 µl of extraction buffer (100 mM Tris; 1 mM KCl; 10 mM EDTA; pH 9.5) is added and after the tube is agitated to resuspend the powdered tissue it is incubated at 65°C for 1 hour, followed by centrifugation for 1 minute at 1500 RPM. 200µl of precipitation buffer (5 M KAc; pH 7.0) is added and the tube is agitated to thoroughly mix, followed by centrifugation at 3000 RPM for 10 minutes. 600 µl of supernatant containing DNA is transferred to a new tube containing 500 µl isopropanol, mixed, and left at room temperature for 10 minutes. The tube is centrifuged for 5 minutes at 3000 RPM to pellet the DNA and supernatant is removed. The DNA pellet is dried at 65°C for 30 minutes, and then 200 µl of TE (10 mM Tris; 1 mM EDTA) buffer is added and incubated at room temperature for at least 30 minutes. The pellet is agitated for 1 minute, centrifuged for 1 minute at 1000 RPM, and stored at 4°C.

Following template DNA extraction, TAQMAN^{™} PCR multiplex amplification reaction is performed on each sample. 3µl of extracted template DNA is mixed with 0.2 µl WT VIC^{™} internal control probe [SEQ ID NO:7] suspended in 18 megohm purified water [Sigma Catalog No. W-4502] (0.2 µM final concentration); 0.2 µl Event 6-FAM^{™} MGB probe [SEQ ID NO:6] suspended in 18 megohm purified water (0.2 µM final concentration); 0.5 µl of zygosity probe primer mix suspended in 18 megohm purified water (1.0 µM final concentration prepared by suspending each primer (SEQ ID NO:3, SEQ ID NO:4, and SEQ ID NO:5) in 18 megohm water at a concentration of 20 µM; and 5 µl of 2X universal master buffer mix [Applied Biosystems part No. 4304437]. Final volume is adjusted to 10 µl with 18 megohm water, and PCR is performed in an Applied Biosystems GeneAmp PCR System 9700 or an MJ Research DNA Engine PTC-225 thermal cycler by using the following parameters:1 cycle 50°C, 2 minutes; 1 cycle 95°C, 10 minutes; 10 cycles of 95°C, 15 seconds, followed by 64°C, 1 minute, -1°C/cycle; 30 cycles of 95°C, 15 seconds, followed by 54°C, 1 minute; and 1 cycle of 10°C, hold temperature.

The following positive controls are used: template DNA from known homozygous 40-3-2 transgenic soybean; template DNA from known hemizygous 40-3-2 transgenic soybean. The following negative controls are used: template DNA from known non-transgenic soybean; no template DNA control.

FAM probe fluorescence is read at 520 nM; VIC^{™} probe fluorescence is read at 550 nM. Fluorogenic MGB TAQMAN^{™} probes are PB65 (6FAM-CCTTTTCCATTTGGG; SEQ ID NO:6) and PB1172 (VIC^{™}- ACCTCGTTTCTATGCTAATTAC; SEQ ID NO:7).

Following crossing of a 40-3-2 line with a conventional line, progeny lines are identified that lack a detectable amplified insert sequence, and are screened for glyphosate sensitivity and traits of interest. Marker-assisted breeding may be employed in this process. The presence of a trans gene may also be assessed in progeny by means of other methods such as other PCR-based methods, Southern blots, northern blots, western blots, or ELISA analysis.

### Example 2: Transgenic progeny of 40-3-2 that comprise a different glyphosate tolerance transgene, and do not contain the functional 40-3-2 insertion.

An elite soybean cultivar comprising ROUNDUP READY® event 40-3-2 may be crossed with a soybean cultivar comprising a different transgenic event that confers glyphosate tolerance, and segregation of the two transgenic inserts and additional agronomic traits is followed. The PCR-based zygosity test for sequences specific to event 40-3-2 is performed as described in Example 1. Similar zygosity testing may optionally be performed to identify progeny homozygous or hemizygous for the other transgenic event or events. Progeny are screened by the PCR-based zygosity test for loss of the 40-3-2 insertion, and selected for glyphosate resistance and other traits of interest.

The methods used to identify heterozygous from homozygous progeny containing 40-3-2 insertion DNA are described in a zygosity assay for which examples of conditions are described in Table 2 and Table 3. The DNA primers used in the zygosity assay are primers (SEQ ID NO:3), (SEQ ID NO:4), (SEQ ID NO:5), 6FAM™ labeled primer (SEQ ID NO:6) and VIC™ labeled primer (SEQ ID NO:7), 6FAM and VIC are florescent dye products of Applied Biosystems (Foster City, CA) attached to the DNA primer.

SEQ ID NO:3, SEQ ID NO:4 and SEQ ID NO:5 when used in these reaction methods produce one DNA amplicon for non-transgenic soybean, two DNA amplicons for heterozygous soybean containing event 40-3-2 DNA, and one DNA amplicon for homozygous 40-3-2 soybean plant. The controls for this analysis should include a positive control from homozygous and heterozygous soybean containing event 40-3-2 DNA, a negative control from non-transgenic soybean, and a negative control that contains no template DNA. This assay is optimized for use with a Stratagene Robocycler, MJ Engine, Perkin-Elmer 9700, or Eppendorf Mastercycler Gradient thermocycler. Other methods and apparatus known to those skilled in the art that produce amplicons that identify the zygosity of the progeny of crosses made with 40-3-2 plants is within the skill of the art.

**Table 2.**

| Zygosity assay reaction solutions | | | |
|---|---|---|---|
| **Step** | **Reagent** | **Amount** | **Comments** |
| 1 | Nuclease-free water | add to 10 µl final volume | - |
| 2 | 2X Universal Master Mix (Applied Biosystems cat. # 4304437) | 5 µl | 1 X final concentration |
| 3 | Primers SEQ ID NO:3, 4, and 5 (resuspended in nuclease-free water to a concentration of 20 µM) | 0.5 µl | 0.25 µM final concentration |
| 4 | Primer 6FAM™ (resuspended in nuclease-free water to a concentration of 10 µM) | 0.2 µl | 0.4 µM final concentration |
| 5 | Primer VIC™ (resuspended in nuclease-free water to a concentration of 10 µM) | 0.2 µl | 0.15 µM final concentration |
| 6 | *REDTaq* DNA polymerase (1 unit/µl) | 1.0 µl (recommended to switch pipets prior to next step) | 1 unit/reaction |
| 7 | Extracted DNA (template): | 3.0 µl | Diluted in water |
| | • Samples to be analyzed (individual • leaves) | 4-80 ng of genomic DNA | |
| | • Negative control | • 4 ng of non-transgenic soybean genomic DNA | |
| | • Negative control | • no DNA template (solution in which DNA was resuspended) | |
| | • Positive control | • 4 ng of genomic DNA from known event 40-3-2 heterozygous soybean | |
| | • Positive control | • 4 ng of genomic DNA from known event 40-3-2 homozygous soybean | |
| 8 | Gently mix, add 1-2 drops of mineral oil on top of each reaction. | | |

Zygosity assay thermocycler conditions: Proceed with the DNA amplification in a Stratagene Robocycler, MJ Engine, Perkin-Elmer 9700, or Eppendorf Mastercycler Gradient thermocycler using the following cycling parameters. When running the PCR in the Eppendorf Mastercycler Gradient or MJ Engine, the thermocycler should be run in the calculated mode. When running the PCR in the Perkin-Elmer 9700, run the thermocycler with the ramp speed set at maximum.

**Table 3.**

| **Cycle No.** | **Settings: Stratagene Robocycler** | |
|---|---|---|
| 1 | 94°C | 3 minutes |
| 38 | 94°C | 1 minute |
| | 60°C | 1 minute |
| | 72°C | 1 minute and 30 seconds |
| 1 | 72°C | 10 minutes |
| | | |

| **Cycle No.** | **Settings: MJ Engine or Perkin-Elmer 9700** | |
|---|---|---|
| 1 | 94°C | 3 minutes |
| 38 | 94°C | 30 seconds |
| | 60°C | 30 seconds |
| | 72°C | 1 minute and 30 seconds |
| 1 | 72°C | 10 minutes |
| | | |

| **Cycle No.** | **Settings: Eppendorf Mastercycler Gradient** | |
|---|---|---|
| 1 | 94°C | 3 minutes |
| 38 | 94°C | 15 seconds |
| | 60°C | 15 seconds |
| | 72°C | 1 minute and 30 seconds |
| 1 | 72°C | 10 minutes |

### Example 3: Conversion of a GA21 event-containing corn line - segregant analysis

The present invention may be applied to corn breeding. An inbred corn line comprising event GA21 was crossed to another inbred line comprising event NK603, and segregation of progeny was followed in order to efficiently identify progeny that lack sequences associated with the GA21 event, comprise the NK603 event, and exhibit other DNA markers of the original parent line that comprises GA21.

Conversion of the GA21 line was performed by means of a multi-tiered marker-assisted breeding approach of event-specific PCR-based assays, linked-marker PCR, and Southern blot analysis, to confirm the presence of event NK603-related DNA sequences and absence of event GA21-related DNA sequences. Following multiple backcrosses, we confirmed the presence of the NK603 event, and of DNA markers associated with the genetic background and agronomic qualities of the original parent that comprises GA21.

The recurrent parent corn inbred line RR728-18GA21 was crossed with donor parent 9034(5)NK603, and backcrossed to the recurrent parent. The BC1S0 was crossed to the recurrent parent, and its genotype was screened by PCR-based assay. Lines exhibiting probable heterozygosity at both transgenic loci were selected and ranked according to the number of PCR markers present for the recurrent parent (RP). Selected lines (BC2S0 generation) were backcrossed to the recurrent parent again (BC3S0), selfed, and screened by Taqman PCR. Of 558 lines sampled, 165 comprised the NK603 event, and 38 of those 165 were deemed highly probable as being heterozygous for GA21 based on the GA21 event assay and co-dominant linked markers. This generation was selfed to yield a BC3F2 generation, and progeny were again screened with respect to GA21-linked PCR markers, NK603 PCR-based zygosity assay, and percentage of recurrent parent markers (PCR-based). Lines selected for further breeding at this stage comprised up to almost 95% recurrent parent markers, were heterozygous for GA21, and comprised NK603 (table 4).

**Table 4**

| Line | % Recurrent Parent Markers |
|---|---|
| 304 | 90.89 |
| 312 | 89.78 |
| 323 | 91.94 |
| 336 | 94.94 |
| 397 | 90.72 |
| 406 | 92.77 |

The BC3F2 generation was selfed, and progeny were grown and selected for tolerance to ROUNDUP®; marker-assisted selection for presence of homozygous null GA21 and homozygous NK603, and highest percentage of recurrent parent markers. 15 out of 251 screened lines were homozygous null GA21 (i.e. lack the GA21 event) and homozygous for the NK603 event. Sixty pools of 5 progeny each of selected lines were subjected to Southern blot analysis of EcoRV-digested genomic DNA. Control lanes included genomic DNA from known GA21 and NK603 corn lines, and a sample of GA21 genomic DNA spiked 1:9 into NK603 genomic DNA to simulate the presence of a hemizygous GA21-containing plant in a five plant pool.

A rice actin promoter probe was used. The probe was prepared by PCR synthesis using pDPG434 as template DNA, and primers ract-F TCGAGGTCATTCATATGCTTGAGAAG [SEQ ID NO:8] and ract-R AAGCTCCGCACGAGGCTGCATTTG [SEQ ID NO:9] followed by digoxigenin labeling. pDPG434 is the plasmid construct used to give rise to the GA21 event (U.S. Patent 6,040,497, incorporated herein by reference), and contains elements in common with the plasmid construct used to give rise to the NK603 event. The probe is a 1.4 Kb DNA fragment spanning the rice actin1 promoter, intron, and 5' untranslated region (UTR). With this probe, NK603-containing genomic DNA yields a hybridizing band of 4 Kb, and GA21-containing genomic DNA yields a hybridizing band of about 21 Kb. The resulting hybridization pattern indicated detectable NK603-specific signal and no detectable GA21-specific signal in experimental lanes. Control lanes yielded expected size GA21 and NK603 specific signals, confirming that the assay was sensitive enough to identify a single hemizygous GA21 individual in a pool of five plants. The converted inbred line is identified as 9034 (NK603).

This method may also be used to identify non-transgenic progeny (i.e. null segregants for both transgenic events) in a cross such as that described above. Such null segregants would, in this case, comprise neither the GA21 nor NK603 events, but may comprise genetic markers and agronomic qualities of either or both parent lines.

All of the methods disclosed and claimed herein can be made and executed without undue experimentation in light of the present disclosure. While the methods of this invention have been described in terms of preferred embodiments, it will be apparent to those of skill in the art that variations may be applied in the steps or in the sequence of steps of the methods described herein without departing from the concept, spirit and scope of the invention. All such similar substitutes and modifications apparent to those skilled in the art are deemed to be within the spirit, scope and concept of the invention.

### REFERENCES

The following references, to the extent that they provide exemplary procedural or other details supplementary to those set forth herein, are specifically incorporated herein by reference:
US Patents 6,825,400; 6,818,807; 6,468,747; 6,563,026; 6,040,497
Hare & Chua, Nature Biotechnol. 20:575-580
Padgette, Crop Sci. 35:1451-1461
Windels, Eur Food Res. Technol. 213:107-112
Bubner, BMC Biotechnol. 4:14 (2004)
Schmidt, Plant Cell Rep. 20:422-428
Song, Plant Cell Rep. 20:948-954
Bubner, Plant Cell Rep. 23:263-271
Windels, Med. Fac. Landbouww Univ. Gent. 64/5b:459-462
Fehr, W.R. 1987. Principles of Cultivar Development, Vol. 1: Theory and Technique*.*
Simmonds, N.W. and J. Smartt. 1999. Principles of Crop Improvement. 2nd Ed.

A method for developing an elite crop variety, comprising:
a) crossing a first elite line comprising a transgene that encodes a useful trait, and which exhibits at least one additional elite trait, with a second line that exhibits a useful trait;
b) obtaining individual F1 hybrid lines;
c) selecting at least one F1 hybrid individual that exhibits a useful trait derived from the first or second line;
d) deriving at least one further progeny generation from the selected F1 hybrid individual;
e) screening F2 or later progeny exhibiting at least one of the additional elite traits of the first line for the presence of the transgene; and
f) identifying at least one individual lacking the transgene of the first line.

The method as defined above, wherein the screening process of step (e) comprises a zygosity assay or ELISA assay.

The method as defined above, wherein the individual identified in step (f) further comprises the useful trait of the second line.

The method as defined above, wherein the useful trait is herbicide tolerance.

The method as defined above, wherein the useful trait is glyphosate tolerance.

The method as defined above, wherein the glyphosate tolerance is provided by expression of glyphosate tolerant CP4 EPSPS, EPSPS, GOX, or GAT

The method as defined above, wherein the useful traits exhibited by the first and second lines both comprise herbicide tolerance.

The method as defined above, wherein the crop is selected from the group consisting of soybean, corn, cotton, rice, wheat, canola, alfalfa, turfgrass, flax, sugar beet, potato, and chicory.

The method as defined above, wherein the crop is soybean and the first elite line comprises glyphosate tolerant soybean event 40-3-2.

The method as defined above, wherein the crop is corn and the first elite line comprises event GA21.

The method as defined above, wherein the second line comprises a transgene.

The method as defined above further comprising:
g) crossing the at least one individual of step (f) with a glyphosate tolerant line to obtain at least one individual F1 plant;
h) selecting at least one F1 individual plant exhibiting glyphosate tolerance;
i) deriving at least one further generation from the F1 individual;
j) screening the further generation for the presence of the additional elite traits of the first line and glyphosate tolerance; and
k) selecting progeny seeds or plants wherein the seeds or plants exhibit glyphosate tolerance.

The method as defined above, wherein the glyphosate tolerance is provided by expression of a CP4 EPSPS, EPSPS, GOX, or GAT.

The method as defined above, wherein the crop is soybean and the glyphosate tolerant line of step (a) is soybean event 781.

A method for producing a non-transgenic elite crop variety, comprising:
a) crossing an elite line, comprising a transgene and exhibiting one or more transgenically-derived elite traits and one or more conventionally-derived elite traits, with a conventional non-transgenic line;
b) obtaining individual F1 hybrid lines;
c) selecting at least one F1 hybrid individual exhibiting at least one elite characteristic of the elite line;
d) deriving at least one further progeny generation from the selected F1 hybrid individual;
e) screening progeny of the hybrid individual exhibiting the at least one conventionally-derived elite traits of the elite line for the presence of the transgene; and
f) selecting progeny exhibiting the at least one conventionally-derived elite traits of the elite line wherein no transgene DNA sequences are present in the progeny genome.

The method as defined above, wherein the elite line comprises soybean event 40-3-2.

The method as defined above, wherein the elite line comprising soybean event 40-3-2 has been exposed to a mutagenizing agent to develop a unique genetic profile.

The method as defined above, wherein the elite line exhibits one or more traits selected from the group consisting of: herbicide tolerance, insect resistance, and male sterility.

The method as defined above, further comprising:
g) crossing the at least one progeny of step (f) with another line to obtain at least one individual F1 plant;
h) selecting at least one F1 individual plant exhibiting one or more elite traits derived from either of its parents;
i) deriving at least one further generation from the F1 individual;
j) screening the further generation for the presence of elite traits; and
k) selecting progeny seeds or plants wherein the seeds or plants exhibit one or more elite traits.

A method for identifying null segregants for a transgene in a plant breeding program, comprising:
a) crossing a first line containing a transgene that encodes a useful trait inserted into its genome with a second line;
b) obtaining F1 hybrid individuals;
c) deriving at least one further progeny generation from the F1 individual;
d) performing zygosity analysis on the further progeny generation, and optionally Southern or western analyses;
e) selecting progeny wherein elements of the transgene are absent and the insertion site of the transgene is restored to approximate its native state, and
f) deriving further generations of the selected progeny,
wherein the removal of the transgene is not caused by the presence of a transgenic recombinase.

The method as defined above, wherein the transgene encodes herbicide tolerance.

The method as defined above, wherein the transgene encodes glyphosate tolerance.

The method as defined above, wherein the transgene encodes CP4 EPSPS, EPSPS, GOX, or GAT.

The method as defined above, wherein the transgene encodes an insecticidal toxin derived from *Bacillus thuringiensis.*

A plant, a progeny plant, or a seed produced by the method as defined hereinbefore.

A plant or seed produced by the method as defined hereinbefore, wherein the plant comprises a transgene.

A transgenic plant or seed as defined above which contains a heterologous gene which encodes an EPSPS enzyme having a Kₘ for phosphoenolpyruvate (PEP) between 1 and 150 µM and a Kᵢ (glyphosate)/Kₘ (PEP) ratio between about 2 and 500, said plant exhibiting tolerance to N-phosphonomethylglycine herbicide at a rate of 1 lb/acre without significant yield reduction due to herbicide application.

A plant or seed produced by the method as defined hereinbefore, wherein the plant does not comprise a transgene.

A plant, seed, progeny plant or progeny seed comprising agronomically elite traits, wherein said agronomically elite traits are derived from a donor line comprising said agronomically elite traits and at least one transgenic insertion, and wherein said plant, seed, progeny plant or progeny seed is further characterized by the absence of the one or more transgenic insertions of the donor line.

The plant as defined above, wherein one of the elite traits is herbicide tolerance.

The plant as defined above, wherein the elite trait is glyphosate tolerance.

The plant as defined above wherein the glyphosate tolerance is provided by expression of glyphosate tolerant CP4 EPSPS, EPSPS, GOX, or GAT.

The method as defined hereinbefore, wherein the transgene encoding CP4 EPSPS comprises a plant DNA virus promoter; a chloroplast transit peptide, CP4 EPSPS; and a NOS terminator.

## Claims

1. A plant or a seed produced by a method comprising:
a) crossing a first elite line comprising a transgene that encodes a useful trait in said first elite line, and which exhibits at least one additional elite trait, with a second line that exhibits a useful trait;
b) obtaining individual F1 hybrid lines;
c) selecting at least one F1 hybrid individual that exhibits a useful trait derived from the first elite line or second line;
d) deriving at least one further progeny generation from the selected F1 hybrid individual;
e) screening F2 or later progeny exhibiting at least one of the additional elite traits of the first elite line for the presence of the transgene;
f) identifying at least one individual as screened in step (e) that lacks the transgene of the first elite line; and
g) crossing the individual identified in step (f) with another line to obtain at least one individual F1 plant.

2. The plant or seed of claim 1, wherein said method further comprises:
h) selecting at least one individual F1 plant obtained in step (g) that exhibits one or more elite traits derived from either of its parents;
i) deriving at least one further generation from the F1 individual selected in step (h);
j) screening the further generation for the presence of elite traits; and
k) selecting at least one individual from the generation screened in step (j) that exhibits one or more elite traits.

3. The plant or seed of claim 1 or 2, wherein the plant or seed comprises a transgene other than the transgene that was segregated out through the crossing steps of said method.

4. The transgenic plant or seed of claim 3, wherein said plant or seed comprises a heterologous gene which encodes an EPSPS enzyme having a Kₘ for phosphoenolpyruvate (PEP) between 1 and 150 µM and a Kᵢ (glyphosate)/Kₘ (PEP) ratio between about 2 and 500, and wherein said plant exhibits tolerance to N-phosphonomethylglycine herbicide at a rate of 1 lb/acre without significant yield reduction due to herbicide application.

5. A plant or seed produced by a method comprising:
a) crossing a first line containing a transgene that encodes a useful trait in said first line with a second line;
b) obtaining an F1 hybrid individual;
c) deriving at least one further progeny generation from the F1 individual;
d) performing zygosity analysis on the further progeny generation, and optionally Southern or western analyses;
e) selecting progeny that do not have the transgene; and
f) deriving further generations of the progeny selected in step (e),
wherein removal of the transgene from the selected progeny is not caused by the presence of a transgenic recombinase.

6. A plant or seed comprising agronomically elite traits, wherein said agronomically elite traits are derived from a donor line comprising said agronomically elite traits and at least one transgenic insertion, and wherein said plant or seed is further **characterized by** the absence of said transgenic insertion of the donor line.

7. The plant of claim 6, wherein one of the elite traits is herbicide tolerance.

8. The plant of claim 7, wherein the elite trait is glyphosate tolerance.

9. The plant of claim 8, wherein the glyphosate tolerance is provided by expression of glyphosate tolerant CP4 5-enolpyruvylshikimate-3-phosphate synthase (EPSPS), EPSPS, glyphosate oxidoreductase (GOX), or glyphosate-N-acetyl transferase (GAT).


SEQUENCE LISTING

<110>  Monsanto Technology LLC

<120>  Development Of Novel Germplasm Using Segregates From
       Transgenic  Crosses

<130>  091820ep

<150>  EP 06787991.6
<151>  2006-07-19

<150>  US 60/703,917
<151>  2005-07-25

<160>  9

<170>  Patentin Version 3.3

<210>  1
<211>  291
<212>  Dna
<213>  Artificial Sequence

<220>
<223>  Sequence Comprising 5' Flanking Sequence Of Primary Insert In
       Soybean Event 40-3-2

<400>  1
CGTGGGTGGG GTCCATCTTT GGGACCCTGT CGGCAGAGGC ATCTTCAACG ATGGCCTTTC      60

CTTTATCGCA ATGATGGCAT TTGTAGGAGC CACCTTCCTT TTCCATTTGG GTTCCCTATG     120

TTTATTTTAA CCTGTATGTA TGATCTTATT TTGAATGAAA TGCAATAAGT TATTTCTAGT     180

AAAAAAAAAT AAACATTTGA TAGAAACAAA TTAAAGCATG CAAAAATAAC TCATTAGCAT     240

CGGTTAAATT GAAGGGTTTG AATAATTTGC ACAAGGTTCT GAATTCAAAT C              291

<210>  2
<211>  860
<212>  Dna
<213>  Artificial Sequence

<220>
<223>  Sequence Comprising 3' Flanking Sequence Of Primary Insert In
       Soybean Event 40-3-2

<400>  2
TTTCTGTTGA ATACGTTAAG CATGTAATAA TTAACATGTA ATGCATGACG TTATTTATGA      60

GATGGGTTTT TATGATTAGA GTCCCGCAAT TATACATTTA ATACGCGATA GAAAACAAAA     120

TATAGCCGCG CAAACTAGGA TAAATTATCG CGCGCGGTGT CATCTATGTT ACTAGATCGG     180

GGATCGATCC CCCACCGGTC CTTCATGTTC GGCGGTCTCG CGAGCGGTGA AACGCGCATC     240

ACCGGCCTTC TGGAAGGCGA GGACGTCATC AATACGGGCA AGGCCATGCA GGCCATGGGC     300

GCCAGGATCC GTAAGGAAGG CGACACCTGG ATCATCGATG GCGTCGGCAA TGGCGGCCTC 360

CTGGCGCCTG AGGCGCCGCT CGATTTCGGC AATGCCGCCA CGGGCTGCCG CCTGACCATG 420

GGCCTCGTCG GGGTCTACGA TTTCAAGCGC ATCATGCTGG GAAATTTTAG CGAGATTATA 480

AGTATCTTCC TGGGGATCTC TGCTGTTACT GGTGAATAGT GAGACAGAGT CTTCTGAGCT 540

CATAGGATAA AATAAATTAT AATTAGTAAA TTTTTTAATT AAATAAATCA ATTACTTCAT 600

AAATAATTTT TTTTATAGAA TATGTTGACA TTCTAGCTGG ATATAGAACT AATATAAAGA 660

AACCTTAAAA ATTTTGTTTG GAAGAATATG TTATTGAAAG ACAAATCTAA TTAAGTTTAT 720

CAGGGTCATT TGTTGAAGAT AGGAAACCTT CAGCAATTTG AATATTAAGT AACTGCTTCT 780

CCCAGAATGA TCGGAGTTTC TCCTCCTGCT ATTACATGAA AAAAAATAAA AAATAAAAAA 840

AAGATAAGAT TAAGCTTCAA 860

<210> 3
<211> 21
<212> Dna
<213> Artificial Sequence

<220>
<223> Sq1147

<400> 3
GGCATTTGTA GGAGCCACCT T 21

<210> 4
<211> 31
<212> Dna
<213> Artificial Sequence

<220>
<223> Sq1148

<400> 4
TTCATTCAAA ATAAGATCAT ACATACAGGT T 31

<210> 5
<211> 23
<212> Dna
<213> Artificial Sequence

<220>
<223> Sq3523

<400> 5
GTGTCGTTCA CATCTTTGCA AGA 23

<210> 6
<211> 15
<212> Dna

<213>  Artificial Sequence

<220>
<223>  Fam Taqman Probe Pb65

<400>  6
CCTTTTCCAT TTGGG                                                        15


<210>  7
<211>  21
<212>  Dna
<213>  Artificial Sequence

<220>
<223>  Vic Taqman Probe Pb1172

<400>  7
ACCTCGTTTC TAGCTAATTA C                                                 21


<210>  8
<211>  30
<212>  Dna
<213>  Artificial Sequence

<220>
<223>  Ract-F Primer

<400>  8
TCGAGGTCAT TCATTCATAT GCTTGAGAAG                                        30


<210>  9
<211>  24
<212>  Dna
<213>  Artificial Sequence

<220>
<223>  Ract-R Primer

<400>  9
AAGCTCCGCA CGAGGCTGCA TTTG                                              24